# EUROPEAN PATENT APPLICATION

(11) **EP 4 108 084 A1**
(43) Date of publication of application: **28.12.2022**
(21) Application number: 20919478.6
(22) Date of filing: 17.02.2020
(51) Int. Cl.: A01N 1/02, C12N 5/02, C12N 5/07, C12Q 1/04

(54) **SAMPLE PRESERVATION COMPOSITION**

(71) Applicant: Nippon Becton Dickinson Company, Ltd., Tokyo 107-0052 (JP); National Cancer Center Japan, Tokyo 104-0045 (JP)
(72) Inventor: NISHIKAWA Hiroyoshi, Tokyo 104-0045 (JP); KOBAYASHI Tamiyo, Tokyo 107-0052 (JP)
(74) Representative: Bals & Vogel Patentanwälte PartGmbB
(86) International application number: PCT/JP2020/005991
(87) International publication number: WO 2021/166022

(57) **Abstract**

A purpose of the present invention is to provide a composition for preserving a sample, in particular, a composition able to preserve cells or a tissue without causing a change in the state of cells. Another purpose of the present invention is to provide a sample preservation method for preparing a suspension containing a target object from a preserved sample, and a method for analyzing a target object in a sample. The present disclosure provides a sample preservation composition containing albumin and lactic acid or a salt thereof. The concentration of sodium ions in the composition is 50-300 mmol/L. Further provided are a method for preserving a sample using the composition, a method for preparing a sample suspension, and a method for analyzing a target object in the sample suspension.

## Description

### Summary of Invention

The present disclosure relates to a composition for preserving samples. The present disclosure further relates to a sample preservation method, a method for adjusting a sample suspension solution including a target substance from a preserved sample, and a method for analyzing a target substance in a sample suspension solution.

### Background Art

Various molecules are known to be expressed in cells depending on the type of cell, and analyzing the type, function, and expression mode, etc., of molecules expressed by cells is a valuable means for understanding cell interaction and response to drugs, etc.

Immune checkpoint inhibitors, which have received attention as a new cancer treatment method, is a drug which inhibits molecules involved in immune suppression such as PD-1 or CTLA-4 which are expressed in immune cells, thereby boosting the anti-tumor immune response of immune cells, and has been shown to have a strong and sustained tumor retraction effect on various types of cancer, including malignant melanoma and gastric cancer. It is known that there are patients who show an effect (responders) and patients who do not show an effect (non-responders) with respect to immune checkpoint inhibitors. Therefore in clinical settings tests for predicting responsiveness to an immune checkpoint inhibitor (companion diagnostics) are conducted prior to administration. Despite carrying out companion diagnostics, however, the response rate of immune checkpoint inhibitors is said to be 20% to 40%, and it cannot be said that accurate results are always obtained with existing test methods. Giving non-responders drugs incurs not only the economic burden of having to pay expensive medical costs despite the lack of effect, but also psychological and physical burdens accompanying side-effects. Moreover, while new immune checkpoint inhibitors are constantly being developed, insurance coverage for new drugs is putting pressure on national medical expenditures. Accordingly, stratifying responders and non-responders is an important task for reducing patient burden and reducing national medical costs.

One of the reasons companion diagnostics cannot provide accurate results is thought to be the fact that the molecular markers are inappropriate during diagnosis. It is common in current companion diagnostics to test for expression of PD-L1, a tumor cell surface molecule. For instance, Keytruda's (TM) PD-L1 IHC 22C3 pharmDx "Dako" which has been approved as a companion diagnostic drug is an assay kit based on immunohistochemical staining using an anti-PD-L1 mouse monoclonal antibody. At the same time, even though immune cells including lymphocytes and macrophages play an important role in anti-tumor immune response, no testing whatsoever is done to see whether such immune cells are present or whether molecules are expressed in immune cells. Moreover, cases of effectiveness in PD-L1 expression-negative patients have been seen in various types of cancer, and it is becoming clearer that simply testing for PD-L1 in tumor cells is insufficient for predicting the effects of immune checkpoint drugs.

There are various types of immune cells and many molecules exist which are expressed in immune cells, but only one or two markers can be detected at once with immunohistochemical staining which is very limited. Accordingly, in order to make a detailed test of whether various immune cells are present around tumor tissue and the state of expression of molecules in the immune cells, a new analytical method is needed which can detect multiple markers. Multi-color analysis using flow cytometry has attracted attention in recent years but has not yet reached the stage of practical implementation.

One reason given is that there is no way to transport tumor tissue removed from a patient from the clinical setting to the testing facility while keeping it in the state it was in inside the patient's body. Analysis such as flow cytometry is rarely carried out in clinical settings; cells and tissue removed from patients are typically sent to testing facilities for analysis. During transportation, the cells or tissue have to be temporarily preserved using saline solution, a cell preservation solution, or a tissue preservation solution, etc. Patent Literature 1 and Patent Literature 2, for example, disclose a preservation solution for preserving cells or tissue in a living state. By using these preservation solutions, cells and tissue can be preserved alive, but because no thought has been given to preserving cells or tissue while maintaining the state they were in when they were inside the patient's body, changes occur to the expression state, etc., of molecules in cells during transportation. Accordingly, results reflecting the state inside the patient's body cannot be obtained using the kinds of analytical methods which have to be used in testing facilities, making it difficult to carry out accurate diagnosis.

Another reason is that no method has been established for extracting immune cells, etc., as single cells from tissue and other types of samples. Cells have conventionally been extracted by mechanically dispersing a tissue sample using a mixer or homogenizer, etc., and then subjecting it to centrifugation. However, this type of mechanical dispersion frequently results in destruction of or damage to the cells which are to be analyzed, with an insufficient number surviving for analysis, making it difficult to obtain enough undamaged cells.

There is therefore a need for a method for preserving and analyzing samples such as cell or tissue without changing the state of the cells which are the subject of analysis.

### Prior Art Documents

### Patent Literature

Patent Literature 1: Patent No. 4947948, specification
Patent Literature 2: Patent Publication No. 2012-116823 A

### Summary of the Invention

### Problem to be Solved by the Invention

The present disclosure has as an object to provide a composition for preserving a sample, specifically a composition allowing preservation of cells, tissue, or a mixture thereof without changing the state of the cells. Furthermore, the present disclosure has as an object to provide a sample preservation method, a method for preparing a suspension solution including the target substance from the preserved sample, and a method for analyzing the target substance included in the sample.

### Means for Solving the Problem

The invention according to the present disclosure for solving the foregoing problem has the following aspects.
(1) A composition for preserving a sample, including albumin, and lactic acid or a salt thereof, wherein the sodium ion concentration of the composition is 50-300 mmol/L.
(2) The composition described in (1), wherein the albumin is included in an amount of 0.01-2% (w/v).
(3) The composition described in (1) or (2), wherein the lactic acid or salt thereof is included in an amount of 0.1-100 mmol/L.
(4) The composition described in any one of (1) to (3), wherein the albumin in bovine serum albumin (BSA).
(5) The composition described in any one of (1) to (4), wherein the lactic acid or salt thereof is sodium lactate.
(6) The composition described in any one of (1) to (5), including 0.01-10 mmol/L of potassium dihydrogen phosphate, and 0.01-50 mmol/L of disodium hydrogen phosphate.
(7) The composition described in any one of (1) to (6), wherein the composition includes a polysaccharide.
(8) The composition described in (7), wherein the polysaccharide is selected from the group consisting of trehalose, a fructooligosaccharide, indigestible dextrin, and a mixture thereof.
(9) The composition described in any one of (1) to (8), wherein the sample is cells, a tissue, or a mixture thereof.
(10) The composition described in any one of (1) to (9), wherein the sample is taken from a mammal.
(11) The composition described in (10), wherein the mammal is selected from the group consisting of human, dog, rat, and mouse.
(12) A sample preservation method, wherein a sample is immersed in the composition described in any one of (1) to (11).
(13) The preservation method described in (12), wherein a preservation temperature is 0°C to 4°, and the composition is in a non-frozen state.
(14) A method for preparing a sample suspension solution including a target substance from a sample, including a) a step of providing the composition described in any one of (1) to (11), b) a step of immersing the sample in the composition, and c) a step of preparing the sample suspension solution by treating the sample with an enzyme.
(15) The method described in (14), wherein the enzyme is at least one selected from the group consisting of collagenase, elastase, hyaluronidase, dispase, glycosidase, deoxyribonuclease, and trypsin.
(16) The method described in (14) or (15), wherein the target substance is cells.
(17) The method described in (15), wherein the cells include living cells.
(18) The method described in (16) or (17), wherein the cells are tumor infiltrating immune cells.
(19) A method for analyzing a target substance in a sample, including a) a step of providing the composition described in any one of (1) to (11), b) a step of immersing the sample in the composition, c) a step of preparing the sample suspension solution by treating the sample with an enzyme, and d) a step of analyzing the target substance in the sample suspension solution.
(20) The method described in (19), wherein step d) is carried out by at least one selected from the group consisting of, flow cytometry, mass cytometry, gene analysis including single-cell gene analysis, imaging, an immunoassay, mass spectrometry, spectroscopic analysis, and combinations thereof.

### Effects of the Invention

The composition and sample preservation method according to the present disclosure is capable of preserving samples, in particular cells or tissues, while maintaining the state inside the body. Furthermore, with the composition and sample preservation method according to the present disclosure, samples can be preserved for extended periods of time, allowing long distance transportation of samples. Accordingly, it is possible to import samples collected abroad while maintaining the state of the cells.

Furthermore, with the method for preparing a sample suspension solution according to the present disclosure, the target substance included in the sample, in particular target cells, can be made into a suspension solution while maintaining the state inside the body. With the method according to the present disclosure, a sample suspension solution can be maintained under milder conditions than with conventional methods, making it possible to extract a larger number of living cells than conventional dispersion methods, while maintaining the local state in the body.

Furthermore, with the analysis method according to the present disclosure, the target substance included in the sample, in particular target cells, can be analyzed, while maintaining the state inside the body. Because the cells can be analyzed in their state inside the body, it is expected that responsiveness to drugs can be more accurately predicted than conventionally in companion diagnostics relating to immune checkpoint inhibitors, for example.

### Brief Description of the Drawings

Fig. 1 shows results (control) of analyzing tumor tissue using flow cytometry in accordance with the method described in embodiment 9.
Fig. 2 shows results (after 48 hours) of analyzing tumor tissue using flow cytometry in accordance with the method described in embodiment 9.
Fig. 3 shows results (after 72 hours) of analyzing tumor tissue using flow cytometry in accordance with the method described in embodiment 9.

### Embodiments

Embodiments of the invention according to the present disclosure are described below, but the present disclosure is not limited to the following embodiments.

### [Composition for Preserving Samples]

A composition according to the present disclosure is a composition for preserving a sample, including albumin, and lactic acid or a salt thereof, wherein the sodium ion concentration of the composition is 50-300 mmol/L.

The composition according to the present disclosure includes albumin. Albumin is a water-soluble protein included the cells and body fluids of animals and plants, etc. The albumin that can be used in one embodiment of the composition according to the present disclosure includes serum albumin found in vertebrate serum, ovalbumin found in egg whites, milk albumin found in milk, and seed albumin found in plant seeds, etc. The vertebrates include, but are not limited to, humans, cows, horses, and rabbits, etc. In the present disclosure, the albumin is preferably serum albumin, more preferably human-derived, bovine-derived, or equine-derived albumin, and most preferably bovine serum albumin (BSA).

According to one embodiment, albumin is included in the composition at a concentration of 0.01 to 2% (w/v), preferably 0.05 to 1% (w/v), more preferably 0.1 to 0.5% (w/v), and most preferably 0.1 to 0.3% (w/v).

The composition according to the present disclosure comprises lactic acid or a salt thereof. "Lactic acid or a salt thereof" means lactic acid or any lactate salt that dissolves in water to yield lactate ions and cations. Examples of a lactate salt include lithium lactate, sodium lactate, potassium lactate, calcium lactate, ammonium lactate, aluminum lactate, and magnesium lactate, etc. The lactic acid or salt thereof is preferably sodium lactate.

According to one embodiment, the lactic acid or a salt thereof is included in the composition so as to have a lactic acid ion concentration in the composition of 0.1 to 100 mmol/L, preferably 1.0 to 50 mmol/L, more preferably 1.0 to 30 mmol/L, and most preferably 1.0 to 30 mmol/L. It is preferably included in the composition at 3.0 to 20 mmol/L.

Lactic acid has two steric structures, D and L, which are in an enantiomeric relationship, and salts of lactate also have a steric structure corresponding to the structure of lactic acid. Enantiomers refer to stereoisomers whose stereostructures are non-superimposable and which are in an "image-mirror image" relationship. An equal mixture of D and L forms is called a racemic mixture, or a DL form. In the composition according to the present disclosure, L forms and DL forms of lactic acid or salts thereof are preferred, and L forms are more preferred.

The composition according to the present disclosure includes sodium ions at a concentration of 50 to 300 mmol/L, preferably 80 to 250 mmol/L, and more preferably 100 to 200 mmol/L. By adjusting the sodium ion concentration to be within this range, the composition according to the present disclosure can preserve samples, in particular cells or tissues, while maintaining the state in the body.

The sodium ion concentration is adjusted by adding substances that dissolve in water to produce sodium ions. Examples of substances that produce sodium ions include, but are not limited to, sodium hydroxide, sodium chloride, sodium carbonate, sodium nitrate, sodium sulfate, sodium hydrogen sulfate, sodium acetate, sodium oxalate, sodium citrate, sodium dihydrogen phosphate, and dihydrogen phosphate, etc. The substance that produces sodium ions is preferably sodium chloride.

The composition according to the present disclosure may further include potassium dihydrogen phosphate and disodium hydrogen phosphate. According to one embodiment, potassium dihydrogen phosphate is included in the composition at a concentration of 0.01 to 10 mmol/L, preferably 0.05 to 5.0 mmol/L, and more preferably 1.0 to 2.5 mmol/L, and disodium hydrogen phosphate is included in the composition at a concentration of 0.01 to 50 mmol/L, preferably 1.0 to 25 mmol/L, and more preferably 5 to 15 mmol/L.

The composition according to the present disclosure preferably does not include calcium ions. In the presence of calcium ions, cells may have increased expression of some molecules, in particular CTLA-4. Accordingly, with a composition including calcium ions it is difficult to preserve a sample while maintaining the state immediately after collection.

The composition according to the present disclosure may include a polysaccharide. A polysaccharide is a compound formed by dehydration bonding of two or more monosaccharides, substituted derivatives thereof, or mixtures thereof. Polysaccharides include, but are not limited to, disaccharides such as sucrose, lactose, maltose and trehalose; trisaccharides such as raffinose and maltotriose; tetrasaccharides such as acarbose and stachyose; oligosaccharides such as malto-oligosaccharides and fructo-oligosaccharides; and glycogen, starch, dextrin, and others containing more molecules, for example. The polysaccharide is preferably selected from the group consisting of trehalose, a fructo-oligosaccharide, an indigestible dextrin, and mixtures thereof; more preferably trehalose. According to one embodiment, the composition according to the present disclosure includes a polysaccharide at a concentration of 0.01 to 3.0% (w/v), preferably 0.03 to 1.0% (w/v), and more preferably 0.05 to 0.5% (w/v).

According to one embodiment, the sample preserved by the composition according to the present disclosure is selected from cells, tissue, or a mixture thereof.

Cells which can be preserved by the composition according to the present disclosure include, but are not limited to, various cells such as lymphocytes, stem cells, skin cells, mucosal cells, hepatocytes, pancreatic islet cells, nerve cells, chondrocytes, endothelial cells, epithelial cells, bone cells, muscle cells, bone marrow cells, hematopoietic stem cells, and tumor cells. When preserving cells, it is possible to preserve cells immediately after they have been collected from an organism, or to preserve cells after they have been artificially cultured. Cells preserved by the composition according to the present disclosure may be living cells or dead cells, and preferably include living cells.

In the present description, "tissue" refers to a population of many types of cells. Tissues that can be preserved by the composition according to the present disclosure include connective tissue such as epithelial tissue, bone tissue, cartilage tissue, blood tissue; and nerve tissue, muscle tissue, and tumor tissue derived from these tissues. The tissue may be collected from an organism or artificially constituted by culturing cells. The tissue preserved by the composition according to the present disclosure is preferably tumor tissue.

According to one embodiment, the cells, tissue, or mixtures thereof are preferably obtained from an animal, and more preferably from a mammal. Mammals include, but are not limited to, humans, orangutans, chimpanzees, monkeys, horses, cows, pigs, dogs, rabbits, mice, rats, hamsters, and guinea pigs, etc. According to one embodiment, the mammal is preferably selected from the group consisting of humans, dogs, mice, rats, hamsters, and guinea pigs, and more preferably humans.

The composition according to the present application is particularly suited to preservation of tumor tissue collected from a human.

### [Sample Preservation Method]

The present application further provides a method for preserving a sample. Preservation of a sample can be done in any manner that allows the sample to be immersed in the composition according to the present disclosure. For example, immersion of the sample may be performed by placing the collected sample in a container into which the composition has been dispensed, or placing the collected sample in a container and subsequently pouring the composition according to the present disclosure into the container.

In the sample preservation method according to the present disclosure, the preservation temperature is preferably approximately 0°C to approximately 4°C, more preferably approximately 0°C to approximately 1°C, and most preferably more than 0°C and 1°C or less, being a temperature at which the preservation solution and sample do not freeze. If the preservation temperature is lower than 0°C, the sample and composition may freeze and the state of the sample might not be maintained. If the preservation temperature exceeds 4°C, the state of the sample may not be able to be maintained.

The method according to the present disclosure allows samples, in particular collected cells, tissues, or mixtures thereof, to be preserved in the state immediately after collection for extended periods of time, such as 24 hours, 48 hours, or 72 hours.

### [Method for Preparing Sample Suspension]

The present disclosure further provides a method for preparing a sample suspension containing a target substance. A method for preparing a sample suspension includes steps of a) providing the composition according to the present disclosure, b) immersing the sample in the composition according to the present disclosure, and c) treating the sample with an enzyme to prepare the sample suspension.
a) The step of providing the composition according to the present disclosure can be carried out using any method, including dispensing into a lidded container, for example.
b) The step of immersing the sample in the composition according to the present disclosure can be performed using any method that allows the sample to be immersed. The method for immersing the sample follows the description in the [Sample Preservation Method] section.
c) The step of treating the sample with an enzyme to prepare a sample suspension may be carried out by any method known to persons skilled in the art, as long as the enzyme treatment can disperse the sample, in particular cells, tissues or mixtures thereof.

Any enzyme can be used for the enzyme treatment. Examples of enzymes that can be used for the enzyme treatment include, but are not limited to, collagenase, elastase, hyaluronidase, neutral proteases including dispase (registered trademark), glycosidase, deoxyribonuclease (DNase), and trypsin, etc.

Enzyme treatment may also be performed using existing cell dispersion solutions, etc. Existing cell dispersion solutions include, for example, BD Horizon (trademark) Dri Tumor & Tissue Dissociation Reagent (TTDR) available from Becton, Dickinson and Company. The enzyme treatment in the method according to the present disclosure preferably uses TTDR.

The enzyme treatment in the method according to the present disclosure can satisfactorily disperse a sample, especially cells or tissues, under milder conditions than previously. The effect of dispersing cells or tissues by enzyme treatment varies depending on factors such as the type of enzyme used, enzyme concentration, treatment temperature, and treatment time. In particular, the type of enzyme used and the enzyme concentration significantly affect the dispersed state of the tissue. Conventionally, in order to obtain adequate dispersion of cells or tissues, steps such as selecting enzymes that act strongly on the substrate or increasing the enzyme concentration have been taken, but these also reduce cell yield and viability, in addition to increasing the risk of cell dysfunction. Accordingly, it has been difficult to achieve both a high cell viability and adequate dispersion of the sample while maintaining the state of the cells.

However, with the method according to the present disclosure, the sample is satisfactorily dispersed even if conditions in the enzyme treatment such as enzyme type, enzyme concentration, treatment time, and treatment temperature are milder than conventionally, and therefore the viability and yield of cells can be increased, and the cells can be dispersed without changing their state. In conventional preservation solutions, extended immersion of samples, especially cells, tissues or mixtures thereof, causes changes in the state of the cells or tissues, making extended immersion impossible, and accordingly it was not possible to sufficiently disperse the cells or tissue under mild conditions. On the other hand, although not a limitation, the composition according to the present disclosure allows samples to be immersed for an extended period of time, causing samples to be softened as the composition, which is a liquid, adequately permeates the samples. Moreover, since the enzyme easily acts on the interior of a sample and not just the surface, the sample is thought to be thoroughly dispersed by the enzyme treatment under milder conditions than conventionally.

The enzyme treatment can be carried out at any temperature, preferably 1 to 45°C, more preferably 10 to 40°C, and most preferably 15 to 30°C, in order to obtain sufficient dispersion without changing the state of the sample. The enzyme treatment may be carried out while controlling the temperature using a device such as a constant temperature water bath that can keep the temperature constant, but also at room temperature. Room temperature refers to a state free from external heating or cooling.

There is no particular limitation on the enzyme treatment time, which is preferably approximately 1 to approximately 60 minutes, more preferably approximately 5 to 30 minutes, and more preferably about 10-20 minutes, in order to avoid changing the state of the sample and obtain adequate dispersion.

The enzyme treatment may be performed in a still state or while stirring or shaking. In order to cause the enzyme to act evenly on the entire sample, the enzyme treatment is preferably carried out with gentle shaking at an intensity that does not damage the cells.

The step of preparing the sample suspension may include any additional operations such as filtration, centrifugation, and staining.

The sample suspension prepared by the method according to the present disclosure contains the target substance. The target substance can be any substance that can be included in a sample. Examples of the target substance include, but are not limited to, cells, genes, proteins including antibodies, amino acids, chemical substances including neurotransmitters, and so on. According to one embodiment, the target substance is a cell. Cells may be living cells or dead cells, but the target substance preferably includes living cells.

Cells that can be target substances include, for example, immune cells, red blood cells, platelets, tumor cells, stem cells, skin cells, mucosal cells, hepatocytes, pancreatic islet cells, nerve cells, chondrocytes, endothelial cells, epithelial cells, osteocytes, muscle cells, bone marrow cells, hematopoietic stem cells, and the like. Immune cells include lymphocytes including T cells, B cells, and NK cells; granulocytes, including neutrophils, eosinophils, and basophils; dendritic cells; and macrophages. The target substance is preferably immune cells, more preferably tumor-infiltrating immune cells, and most preferably tumor-infiltrating lymphocytes (TIL).

Tumor-infiltrating immune cells refer to immune cells that infiltrate tumor tissue; of these, lymphocytes in particular are referred to as TILs. TILs are known to express cell surface molecules such as CD4, CD8, CTLA-4, and PD-1, etc., depending on the cell type. TILs include CD4-positive (CD4⁺) T cells, CD8-positive (CD8⁺) T cells, regulatory T cells (Treg), and NK cells.

### [Method for Analyzing the Target Substance]

The present disclosure further provides a method for analyzing samples. A method for analyzing samples comprises a) a step of providing the composition described in claims 1-10; b) a step of immersing the sample in the composition; c) a step of treating the sample with an enzyme to form a sample suspension solution; and d) a step of analyzing the target substance in said sample suspension. Steps a) to c) follow the description in the section [Method for Preparing Sample Suspension].

In d), the analyzing step, any analysis method can be used. Analysis methods include, but are not limited to, flow cytometry, mass cytometry, genetic analysis including single cell genetic analysis, imaging, immunoassays, mass spectroscopy, spectroscopy, and combinations thereof. In the method according to the present disclosure, the analysis method is preferably flow cytometry and mass cytometry, and more preferably flow cytometry.

According to the analysis method according to the present disclosure, a sample can be analyzed without changing the target substance contained in the sample, particularly cells, from the state immediately after collection.

The analysis method according to the present disclosure is suited to analyzing cells contained in tissue, particularly for analyzing TILs in tumor tissue using flow cytometry. According to the analysis method of the present disclosure, a larger number of living cells can be analyzed while still in the patient's body. Therefore, the analysis method according to the present disclosure is expected to be useful for accurate prediction of drug responsiveness and patient prognosis in companion diagnostics associated with immune checkpoint inhibitors, for example.

Furthermore, the analysis method according to the present disclosure allows long-distance transportation of samples as the collected samples can be preserved for extended periods of time while maintaining the state immediately after collection. Accordingly, with the method according to the present disclosure, a sample collected at a hospital, for example, can be transported to a distant laboratory for analysis.

### Embodiments

Aspects of the present disclosure are described below in detail with reference to the embodiments, but these are merely illustrations and are not intended to narrow the scope of the present disclosure.

The capabilities of the composition according to the present disclosure were analyzed by labeling cells contained in a sample suspension solution and conducting analysis using flow cytometry to analyze the number of living cells and the percentage of each cell in each cell population. Cells to be analyzed were labeled with the reagents listed in Table 1 for human-derived samples and Table 2 for mouse-derived samples. For human-derived samples, BD Pharmingen (trademark) Human BD Fc Block (trademark) (BD) was added prior to antibody staining to prevent non-specific binding of antibodies. Furthermore, both the human-derived samples and the mouse-derived samples were subjected to cell membrane fixation and permeabilization using BD Pharmingen (trademark) Transcription Factor Buffer Set (BD), followed by antibody staining of intracellular FoxP3 molecules.

After identifying live cells by FVS (Fixable Viability Stain), the results were gated in the order of lymphocytes > CD3⁺ cells > CD4⁺ cells or CD8⁺ cells. CD4⁺ cells which were positive for FoxP3 and negative for CD45RA were designated as Tregs (regulatory T cells). On the other hand, in mouse-derived samples, FoxP3-positive and CD25-positive cells were designated as Tregs (regulatory T cells). Thus, the percentage of each cell refers to the percentage of lymphocytes in the live cell population, the percentage of CD3⁺ cells in the lymphocyte population, the percentage of CD4⁺ or CD8⁺ cells in the CD3⁺ cell population, and the percentage of Tregs in the CD4⁺ cell population. Regarding CD8⁺ cells and Tregs, CTLA-4⁺ and PD-1⁺ cells were gated, respectively. The number of cells per tissue (mg) was calculated using BD Trucount (trademark) Absolute Counting Tubes (Becton, Dickinson and Company, hereinafter also referred to as "BD").

### [Table 1]

**Table 1. Reagents used for cell labeling (human-derived samples)**

| Reagent type | Product name (manufacturer) |
|---|---|
| Reagent for life-or-death determination | BD Horizon (trademark) Fixable Viability Stain 780 (BD) |
| Anti-human CD3 antibody | BD Horizon (trademark) CD3 V500-C (SK7) (BD) |
| Anti-human CD4 antibody | PerCP-Cy (trademark) 5.5 Mouse Anti-Human CD4 (SK3) (BD) |
| Anti-human CD8 antibody | FITC Mouse Anti-Human CD8 (SKI) (BD) |
| Anti-human CD45RA antibody | PE-Cy (trademark) 7 Mouse Anti-Human CD45RA (L48) (BD) |
| Anti-human PD-1 antibody | BD Horizon (trademark) BV421 Mouse Anti-Human CD279 (MIH4) (BD) |
| Anti-human CTLA-4 antibody | BD Pharmingen (trademark) APC Mouse Anti-Human CD152 (BNI 3) (BD) |
| Anti-human FoxP83 substance | BD Pharmingen (trademark) PE Mouse anti-Human FoxP3 (236A/E7) (BD) |

### [Table 2]

**Table 2. Reagents used for cell labeling (mouse-derived samples)**

| Reagent type | Product name (manufacturer) |
|---|---|
| Reagent for life-or-death determination | BD Horizon (trademark) Fixable Viability Stain 780 (BD) |
| Anti-mouse CD3 antibody | BD Pharmingen (trademark) Alexa Fluor (registered trademark) 700 Rat Anti-Mouse CD3 (17A2) (BD) |
| Anti-mouse CD4 antibody | BD Pharmingen (trademark) FITC Rat Anti-Mouse CD4 (GK1.5) (BD) |
| Anti-mouse CD8 antibody | Brilliant Violet 605 (trademark) anti-mouse CD8a Antibody (53-6.7) (BioLegend) |
| Anti-mouse CD19 antibody | Brilliant Violet 711 (trademark) anti-mouse CD19 Antibody (6D5) (BioLegend) |
| Anti-mouse CD25 antibody | Brilliant Violet 785 (trademark) anti-mouse CD25 Antibody (PC61) (BioLegend) |
| Anti-mouse PD-1 antibody | Brilliant Violet 785 (trademark) anti-mouse CD279 (PD-1) Antibody (29F.1A12) (BioLegend) |
| Anti-mouse FoxP3 substance | FOXP3 Monoclonal Antibody, PE, eBioscience (trademark) (FJK-16s) (Thermo Fisher Scientific) |

### (Embodiment 1: BSA Effect Evaluation 1)

Composition 1 and composition 2 were prepared by dissolving the components listed in Table 3 in ultrapure water. Approximately 30 mg (3 pieces: approximately 10 mg/piece) of colon cancer-derived tumor tissue excised from a patient was mixed with approximately 15 mL of the composition and preserved at a temperature of 0°C to 1°C. After 24 hours and 48 hours, the composition was removed by aspirator, 7 mL of TTDR (BD) was added, and the tissue was treated with the enzyme for 15 minutes at room temperature with gentle shaking. The reagents listed in Table 1 were then added to label the cells. After labeling, the sample suspension was washed and analyzed with the BD FACSLyric (trademark) flow cytometer (BD). The control were similarly handled, except that no preservation with the compositions was done.

### [Table 3]

**Table 3. Components in Compositions**

| Component | Composition 1 | Composition 2 |
|---|---|---|
| BSA % (w/v) | | 0.2 |
| L-sodium lactate % (w/v) | 0.1 | 0.1 |
| NaCl mmol/L | 145.1 | 145.1 |
| KCl mmol/L | 2.5 | 2.5 |
| Na ion concentration mmol/L | 154 | 154 |
| Lactic acid ion concentration mmol/L | 8.9 | 8.9 |

The results are shown in Table 4 and represented as the number of live cells per 10 mg of human colon cancer-derived tumor tissue. The results showed that composition 2 containing BSA increased the viability of cells compared to composition 1 having no BSA. Of greater interest was the fact that more live cells were detected in the tissue preserved with composition 2 than in the control.

### [Table 4]

**Table 4. Number of Cells (number per 10 mg of tissue)**

| Type of cell | Control | Composition 1 | | Composition 2 | |
|---|---|---|---|---|---|
| | | 24 hr | 48 hr | 24 hr | 48 hr |
| Lymphocytes | 174655 | 80258 | 243920 | 324279 | 533755 |
| CD3⁺ cells | 48642 | 15249 | 50979 | 125496 | 145715 |
| CD4⁺ cells | 33286 | 8570 | 33238 | 81321 | 103021 |
| Treg | 7561 | 2657 | 10038 | 19192 | 24210 |
| CD8⁺ cells | 10873 | 5063 | 12337 | 27735 | 28997 |
| CTLA-4⁺ Treg | 1141 | 194 | 2690 | 9201 | 4145 |
| PD-1⁺ Treg | 3153 | 983 | 4668 | 8847 | 11064 |
| PD1⁺CD8⁺ cells | 3159 | 1620 | 4676 | 7128 | 8380 |

### (Embodiment 2: BSA Effect Evaluation 2)

Compositions 3 to 5 were prepared by dissolving the components listed in Table 5 in ultrapure water. Next, 1×10⁶ PBMCs from healthy subjects were mixed with approximately 5 mL of the compositions and preserved at a temperature of 0°C to 1°C. After 24 hours, the reagents listed in Table 1 were added and the cells were labeled. After labeling, the sample suspension solutions were washed and analyzed with a flow cytometer.

### [Table 5]

**Table 5. Components in Compositions**

| Component | Composition 1 | Composition 2 | Composition 3 |
|---|---|---|---|
| BSA % (w/v) | 0.1 | 0.2 | 0.5 |
| L-sodium lactate % (w/v) | 0.5 | 0.5 | 0.5 |
| NaCl mmol/L | 154 | 154 | 154 |
| KCl mmol/L | 198.5 | 198.5 | 198.5 |
| Na ion concentration mmol/L | 44.5 | 44.5 | 44.5 |
| Lactic acid ion concentration mmol/L | 0.1 | 0.2 | 0.5 |

Table 6 shows the results. The percentages of the cells were basically the same among compositions 3 to 5, among which the amount of BSA added was varied.

### [Table 6]

**Table 6. Percentages (%) of each type of cell**

| Type of cell | Composition 3 | Composition 4 | Composition 5 |
|---|---|---|---|
| CD4⁺ Treg | 0.52 | 0.52 | 0.55 |
| CTLA-4⁺ CD8⁺ cells | 0.011 | 0.011 | 0.23 |
| PD-1⁺ Treg | 8.00 | 11.9 | 9.55 |
| PD1⁺ CD8⁺ cells | 16.8 | 19.6 | 17.1 |

### (Embodiment 3: Lactic Acid Ion Concentration Effect Evaluation)

Compositions 6 to 9 were prepared by dissolving the components shown in Table 7 in ultrapure water. Then approximately 30 mg of colon cancer-derived tumor tissue (3 pieces: approximately 10 mg/piece) excised from the patient was added to approximately 15 mL of composition 6, and about 30 mg of lung cancer-derived tumor tissue (3 pieces: approximately 10 mg/piece) was added to approximately 15 mL each of compositions 7 to 9, immersed, and preserved at a temperature of 0°C to 1°C. After 24 hours, the compositions were aspirated off, 7 mL of TTDR was added, and the tissue was treated with the enzymes for 15 minutes at room temperature with gentle shaking. The reagents listed in Table 1 were then added and the cells were labeled. After labeling, the sample suspension solutions were washed and analyzed with a flow cytometer. The control was handled in the same manner, except that the excised tissue was immediately subjected to the enzyme treatment without preservation with a composition.

### [Table 7]

**Table 7. Components in Compositions**

| Component | Composition 6 | Composition 7 | Composition 8 | Composition 9 |
|---|---|---|---|---|
| BSA % (w/v) | 0.2 | 0.2 | 0.2 | 0.2 |
| L-sodium lactate % (w/v) | 0.1 | 0.3 | 0.5 | 1.0 |
| NaCl mmol/L | 145.1 | 145.1 | 145.1 | 145.1 |
| KCl mmol/L | 2 | 1 | 1 | |
| Na ion concentration mmol/L | 154 | 171.8 | 189.6 | 234 |
| Lactic acid ion concentration mmol/L | 8.9 | 26.7 | 44.5 | 88.9 |

The results are shown in Tables 8 to 10. Expression of CTLA-4 was basically the same as the control for all cells. On the other hand, expression of PD-1 tended to increase as L-sodium lactate increased, especially in Tregs.

### [Table 8]

**Table 8. Percentages (%) of each type of cell**

| Type of cell | Control | Composition 6 |
|---|---|---|
| CTLA-4⁺ Treg | 11.5 | 13.4 |
| PD-1⁺ Treg | 57.4 | 63.2 |
| CTLA-4⁺ CD8⁺ cells | 0.2 | 0.4 |
| PD-1⁺ CD8⁺ cells | 44.5 | 46.1 |

### [Table 9]

**Table 9. Percentages (%) of each type of cell**

| Type of cell | Control | Composition 7 | Composition 8 |
|---|---|---|---|
| CTLA-4⁺ Treg | 16.8 | 20.4 | 24.7 |
| PD-1⁺ Treg | 54.8 | 12.1 | 85.0 |
| CTLA-4⁺ CD8⁺ cells | 0.1 | 0.16 | 0.17 |
| PD-1⁺ CD8⁺ cells | 55.3 | 49.9 | 60.8 |

### [Table 10]

**Table 10. Percentages (%) of each type of cell**

| Type of cell | Control | Composition 9 |
|---|---|---|
| CTLA-4⁺ Treg | 6.8 | 1.5 |
| PD-1⁺ Treg | 59.8 | 14.4 |
| CTLA-4⁺ CD8⁺ cells | 0.1 | 0.1 |
| PD-1⁺ CD8⁺ cells | 65.7 | 78.5 |

### (Embodiment 4: Sodium Ion Concentration Effect Evaluation)

Compositions 10 to 13 were prepared by dissolving the components listed in Table 11 in ultrapure water. Approximately 20 mg (2 pieces: approximately 10 mg/piece) of a spleen excised from a mouse was then immersed in approximately 15 mL of the compositions and preserved at a temperature of 0°C to 1°C. After 24 hours, the compositions were aspirated off, 7 mL of TTDR was added, and the tissue was treated with the enzyme for 15 minutes at room temperature with gentle shaking. The reagents listed in Table 2 were then added and the cells were labeled. After labeling, the sample suspension solutions were washed and analyzed with a flow cytometer. The control was treated in the same manner except that the excised tissue was immediately subjected to the enzyme treatment without preservation with a composition.

### [Table 11]

**Table 11. Components in Compositions**

| Component | Composition 10 | Composition 11 | Composition 12 | Composition 13 |
|---|---|---|---|---|
| BSA % (w/v) | 0.2 | 0.2 | 0.2 | 0.2 |
| L-sodium lactate % (w/v) | 0.1 | 0.1 | 0.1 | 0.1 |
| NaCl mmol/L | 41.1 | 91.1 | 145.1 | 241.1 |
| KCl mmol/L | 3.9 | 3.9 | 3.9 | 3.9 |
| Na ion concentration mmol/L | 50 | 100 | 154 | 250 |
| Lactic acid ion concentration mmol/L | 8.9 | 8.9 | 8.9 | 8.9 |

The results are shown below. For all of the compositions, the lymphocytes, the CD4⁺ cells, the Tregs, and the CD8⁺ cells were detected at about the same rate as the control. the CD3⁺ cells detected at about the same rate as the control with composition 9 and composition 10. It was found that expression of PD-1 tended to increase when the sodium ion concentration in the compositions was low.

### [Table 12]

**Table 12. Percentages (%) of each type of cell**

| Type of cell | Control | Composition 9 | Composition 11 | Composition 12 | Composition 13 |
|---|---|---|---|---|---|
| Lymphocytes | 91.0 | 93.7 | 91.9 | 87.9 | 82.0 |
| CD3⁺ cells | 22.5 | 12.3 | 21.0 | 23.8 | 13.5 |
| CD4⁺ cells | 59.5 | 52.2 | 55.5 | 59.5 | 58.9 |
| Treg | 9.27 | 9.43 | 7.92 | 9.01 | 10.1 |
| PD-1⁺ Treg | 20.4 | 33.9 | 22.9 | 20.6 | 21.9 |
| CD8⁺ cells | 33.4 | 34.7 | 34.9 | 33.4 | 32.9 |
| PD-1⁺ CD8⁺ cells | 0.63 | 1.28 | 0.59 | 0.38 | 0.48 |

### (Embodiment 5: Effect of Adding a Polysaccharide)

Compositions 14 to 16 were prepared by dissolving the components listed in Table 13 in ultrapure water. Approximately 30 mg (3 pieces: approximately 10 mg/piece) of a colon cancer-derived tumor tissue excised from a patient was then immersed in 15 mL of the compositions and preserved at a temperature of 0°C to 1°C. After 48 hours, the compositions were aspirated off, 7 mL of TTDR was added, and the tissue was treated with the enzyme for 15 minutes at room temperature with gentle shaking. The reagents listed in Table 1 were then added and the cells were labeled. After labeling, the sample suspension solutions were washed and analyzed with a flow cytometer. The control was treated in the same manner except that the excised tissue was immediately subjected to the enzyme treatment without preservation with a composition.

### [Table 13]

**Table 13. Components in Compositions**

| Component | Composition 14 | Composition 15 | Composition 16 |
|---|---|---|---|
| BSA % (w/v) | 0.2 | 0.2 | 0.2 |
| L-sodium lactate % (w/v) | 0.1 | 0.1 | 0.1 |
| NaCl mmol/L | 145.1 | 145.1 | 145.1 |
| KCl mmol/L | 3.9 | 3.9 | 3.9 |
| Trehalose % (w/v) | 0.1 | | |
| Fructooligosaccharide % (w/v) | | 0.1 | |
| Indigestible dextrin % (w/v) | | | 0.114129 |
| Na ion concentration mmol/L | 154 | 154 | 154 |
| Lactic acid ion concentration mmol/L | 8.9 | 8.9 | 8.9 |

Table 14 shows the results. the percentage of the cells in all of the compositions was about the same as in the control.

### [Table 14]

**Table 14. Percentages (%) of each type of cell**

| Type of cell | Control | Composition 14 | Composition 15 | Composition 16 |
|---|---|---|---|---|
| CTLA-4⁺ Treg | 39.5 | 40.2 | 40.5 | 40.7 |
| PD-1⁺ Treg | 61.4 | 58.1 | 61.0 | 64.9 |
| CTLA-4⁺ CD8⁺ cells | 0.49 | 0.16 | 0.18 | 0.17 |
| PD-1+ CD8⁺ cells | 78.0 | 76.1 | 79.1 | 81.3 |

### (Embodiment 6: Preservation Temperature Effect)

Composition 17 was prepared by adding 58 mL of ultrapure water to 942 mL of GIBCO (registered trademark) D-PBS(-) (Thermo Fisher Scientific, catalog number: 14190) and then adding BSA and L-sodium lactate. Subsequently, approximately 20 mg of spleen (2 pieces: approximately 10 mg/piece) excised from a mouse was immersed in approximately 15 mL of the composition and preserved on ice and at 4°C. After 48 hours, the composition was removed with an aspirator, 7 mL of TTDR was added, and the tissue was treated with an enzyme for 15 minutes at room temperature with gentle shaking. The reagents listed in Table 2 were then added and the cells were labeled. After labeling, sample suspension solutions were washed and analyzed with a flow cytometer. The control was handled the same except that the excised tissue was immediately subjected to the enzyme treatment without preservation with a composition.

### [Table 15]

**Table 14. Components in Compositions**

| Component | Composition 17 |
|---|---|
| BSA % (w/v) | 0.2 |
| L-sodium lactate % (w/v) | 0.1 |
| NaCl mmol/L | 129.9 |
| KH₂PO₄ mmol/L | 1.4 |
| Na₂HPO₄₋₇H₂O mmol/L | 7.6 |
| KCl mmol/L | 2.5 |
| Trehalose % (w/v) | 0.2 |
| Na ion concentration mmol/L | 154 |
| Lactic acid ion concentration mmol/L | 8.9 |

The results are shown in Table 16. A similar number of cells per 10 mg of tissue was detected in tissue stored on ice as in the control, whereas the tissue preserved at 4°C showed a decrease in Treg and CD8⁺ cells. Cell percentages were similar to the control under both conditions.

### [Table 16]

**Table 16. Number of cells (number per 10 mg of tissue)**

| Type of cell | Control | On ice | 4°C |
|---|---|---|---|
| Lymphocytes | 10367684 | 12990957 | 11798198 |
| CD3⁺ cells | 2311782 | 2658375 | 2096065 |
| CD19⁺ cells | 6908669 | 8603636 | 8243534 |
| CD4⁺ cells | 1409716 | 1670482 | 1321392 |
| Treg | 118820 | 129821 | 85645 |
| CD8⁺ cells | 714948 | 852163 | 637407 |
| PD-1⁺ Treg | 32915 | 41095 | 25869 |
| PD1⁺ CD8⁺ cells | 15253 | 14504 | 13785 |

### [Table 17]

**Table 17. Percentages (%) of each type of cell**

| Type of cell | Control | On ice | 4°C |
|---|---|---|---|
| Lymphocytes | 95.8 | 95.6 | 95.3 |
| CD3⁺ cells | 22.3 | 20.5 | 17.8 |
| CD19⁺ cells | 66.7 | 66.2 | 69.9 |
| CD4⁺ cells | 61.0 | 62.8 | 63.0 |
| Treg | 8.4 | 1.8 | 6.5 |
| CD8⁺ cells | 33.6 | 32.1 | 30.4 |
| PD-1⁺ Treg | 21.1 | 31.7 | 30.2 |
| PD1⁺CD8⁺ cells | 2.0 | 1.7 | 2.2 |

### (Embodiment 7: Calcium Ion Effects)

Composition 7 was prepared by dissolving the components shown in Table 7 in ultrapure water. For comparison, a glucose-free RPMI medium (Thermo Fisher Scientific) containing 0.2% BSA was also prepared. Subsequently, 1×10⁶ PBMCs derived from healthy subjects were suspended in approximately 5 mL of the composition or about 5 mL of the RPMI medium and preserved at a temperature of 0°C to 1°C. After 24 hours, the reagents listed in Table 1 were added and the cells were labeled. After labeling, sample suspensions were washed and analyzed with a flow cytometer. The control was handled the same except that the excised tissue was immediately subjected to the enzyme treatment without preservation with a composition.

The results are shown in Table 18. PBMCs preserved in the RPMI medium showed significantly enhanced expression of CTLA-4, probably because the calcium ions contained in the RPMI medium enhanced CTLA-4 expression.

### [Table 18]

**Table 18. Percentages (%) of each type of cell**

| Type of cell | Control | Composition 7 | RPMI medium |
|---|---|---|---|
| CTLA-4⁺ Treg | 1.34 | 0.56 | 3.33 |
| CTLA-4⁺ CD8⁺ cells | 0.006 | 0.004 | 0.005 |

### (Embodiment 8: Performance Evaluation of the Composition According to the Disclosure in Human-Derived Tumor Tissues 1)

Composition 18 was prepared by adding 58 mL of ultrapure water to 942 mL of GIBCO D-PBS(-) and then adding BSA and L-sodium lactate. The components shown in Table 19 were also dissolved in ultrapure water to prepare composition 19. Approximately 30 mg (3 pieces: approximately 10 mg/piece) of colon cancer-derived tumor tissue excised from a patient was then immersed in approximately 15 mL of the compositions and preserved at a temperature of 0°C to 1°C. After 72 hours, the compositions were removed with an aspirator, 7 mL of TTDR was added, and the tissue was treated with an enzyme for 15 minutes at room temperature with gentle shaking. The reagents listed in Table 1 were then added and the cells were labeled. After labeling, the sample suspension solutions were washed and analyzed with a flow cytometer. The control was handled the same except that the excised tissue was immediately subjected to the enzyme treatment without preservation with a composition. The tests were performed with n=4 for the control and n=3 otherwise.

### [Table 19]

**Table 19. Components in Compositions**

| Component | Composition 18 | Composition 19 |
|---|---|---|
| BSA % (w/v) | 0.2 | 0.2 |
| L-sodium lactate % (w/v) | 0.1 | 0.1 |
| NaCl mmol/L | 129.9 | 145.1 |
| KH₂PO₄ mmol/L | 1.4 | |
| Na₂HPO₄₋₇H₂O mmol/L | 1.6 | |
| KCl mmol/L | 2.5 | 3.9 |
| Trehalose % (w/v) | 154 | 154 |
| Na ion concentration mmol/L | 8.9 | 8.9 |
| Lactic acid ion concentration mmol/L | 0.2 | 0.2 |

The results are shown in Tables 20 and 21 as mean and standard deviations (SD). For both compositions, the number of cells per 10 mg of tissue was equal to or greater than the control. Furthermore, the percentage of each type of cell was almost the same as the control in both compositions. Therefore, it was shown that the composition according to the present disclosure can preserve tumor tissue for extended periods of time while maintaining TILs in the state in the tumor.

### [Table 20]

**Table 20. Number of cells (number per 10 mg of tissue)**

| Type of cell | Control | | Composition 18 | | Composition 19 | |
|---|---|---|---|---|---|---|
| | Average | SD | Average | SD | Average | SD |
| Living cells | 75052 | 13703 | 122193 | 51778 | 105083 | 44255 |
| Lymphocytes | 46308 | 13447 | 72585 | 38684 | 69589 | 31088 |
| CD3⁺ cells | 35752 | 13081 | 55089 | 31256 | 51250 | 23451 |
| CD4⁺ cells | 17374 | 5846 | 20402 | 14127 | 21950 | 8701 |
| Treg | 4391 | 1431 | 3611 | 1775 | 4987 | 1292 |
| CD8⁺ cells | 15473 | 6337 | 28726 | 15818 | 24386 | 12095 |
| CTLA-4⁺ Treg | 553 | 116 | 323 | 143 | 609 | 118 |
| PD-1⁺ Treg | 1910 | 683 | 1432 | 699 | 1986 | 478 |
| CTLA-4⁺ CD8⁺ Treg | 92 | 27 | 41 | 23 | 60 | 23 |
| PD-1⁺ CD8⁺ cells | 6638 | 2649 | 8341 | 2482 | 10474 | 3602 |

### [Table 21]

**Table 21. Percentages (%) of each type of cell**

| Type of cell | Control | | Composition 18 | | Composition 19 | |
|---|---|---|---|---|---|---|
| | Average | SD | Average | SD | Average | SD |
| CD3⁺ cells | 75.0 | 8.5 | 72.5 | 6.4 | 13.2 | 1.0 |
| CD4⁺ cells | 49.1 | 3.3 | 36.5 | 7.6 | 44.4 | 3.6 |
| Treg | 25.4 | 12.1 | 21.9 | 6.3 | 24.5 | 4.7 |
| CD8⁺ cells | 42.8 | 2.8 | 54.4 | 11.3 | 46.5 | 2.5 |
| CTLA-4⁺ Treg | 13.1 | 2.1 | 9.1 | 0.8 | 12.5 | 11.9 |
| PD-1⁺ Treg | 42.7 | 3.4 | 40.4 | 3.0 | 40.1 | 1.1 |
| CTLA-4⁺ CD8⁺ Treg | 0.7 | 0.3 | 0.1 | 0.0 | 0.3 | 0.1 |
| PD-1+ CD8⁺ cells | 42.9 | 1.7 | 40.3 | 20.5 | 47.3 | 8.8 |

### (Embodiment 9: Performance Evaluation of the Composition According to the Disclosure in Human-Derived Tumor Tissues 2)

The performance of existing cell preservation solutions and compositions according to the present disclosure were compared. Composition 20 was prepared by adding 58 mL of ultrapure water to 942 mL of GIBCO D-PBS(-) and then adding BSA, L-sodium lactate, and trehalose. Approximately 30 mg (3 pieces: about 10 mg/piece) of colon cancer-derived tumor tissue excised from a patient was immersed in approximately 15 mL of composition 20 or HypoThermosol (registered trademark) (HemaCare) and preserved at a temperature of 0°C to 1°C. After 48 hours and 72 hours, 7 mL of TTDR was added and the tissue was treated with an enzyme for 15 minutes at room temperature with gentle shaking. The reagents listed in Table 1 were then added and the cells were labeled. After labeling, the sample suspension solutionss were washed and analyzed with a flow cytometer. The control was handled the same except that the excised tissue was immediately subjected to the enzyme treatment without preservation with a composition. The tests were carried out with n=3 for the control and 48 hours and n=2 for 72 hours.

### [Table 22]

**Table 22. Components in Compositions**

| Component | Composition 20 |
|---|---|
| BSA % (w/v) | 0.2 |
| L-sodium lactate % (w/v) | 0.1 |
| NaCl mmol/L | 129.9 |
| KH₂PO₄ mmol/L | 1.4 |
| Na₂HPO₄₋₇H₂O mmol/L | 7.6 |
| KCl mmol/L | 2.5 |
| Trehalose % (w/v) | 0.1 |
| Na ion concentration mmol/L | 154 |
| Lactic acid ion concentration mmol/L | 8.9 |

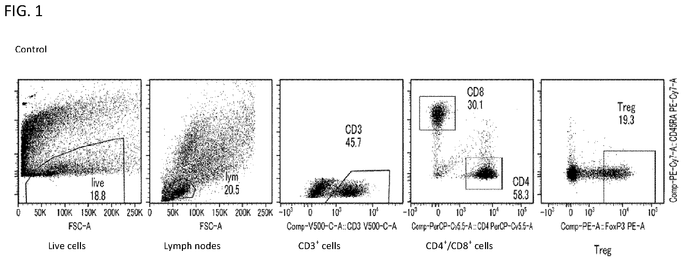

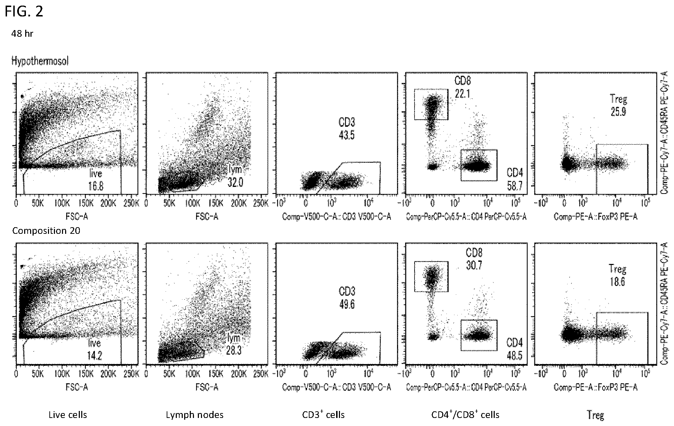

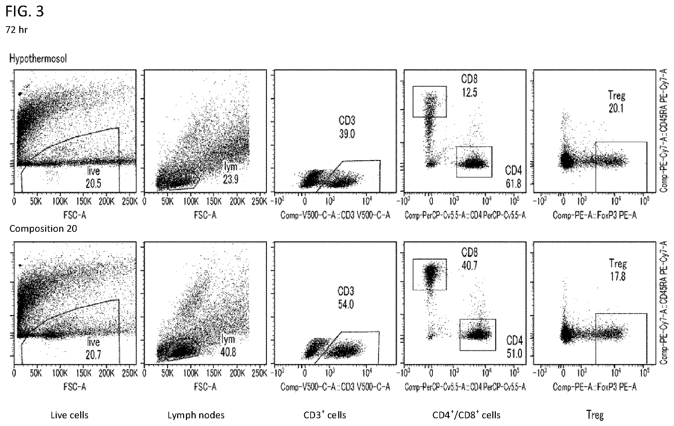

The results are shown in Tables 23 to 26. A larger number of live cells were detected in tissue preserved with composition 20 according to the present disclosure than in tissue preserved with HypoThermosol, indicating that the composition according to the present disclosure can enhance cell viability over existing preservation solutions. Also, as shown in FIG. 1 to FIG. 3, the tissue preserved with HypoThermosol displayed a marked decrease in CD8 expression after 72 hours, whereas the tissue preserved with the composition according to the present disclosure was at the same level as the control. These results thus indicate that the composition according to the present disclosure can preserve tumor tissue while maintaining TIL in the state in the tumor.

### [Table 23]

**Table 23. Number of cells (number per 10 mg of tissue) - 48 hr**

| Type of cell | Control | | Composition 20 | | HypoThermosol | |
|---|---|---|---|---|---|---|
| | Average | SD | Average | SD | Average | SD |
| Living cells | 95833 | 40162 | 128846 | 42330 | 153388 | 31773 |
| Lymphocytes | 25204 | 17928 | 42933 | 18230 | 45404 | 6538 |
| CD3⁺ cells | 13706 | 13052 | 21191 | 9689 | 22172 | 38317 |
| CD4⁺ cells | 7152 | 6501 | 11721 | 6570 | 11505 | 1301 |
| Treg | 1368 | 1251 | 3296 | 2431 | 2689 | 611 |
| CD8⁺ cells | 5447 | 6017 | 5600 | 2409 | 6414 | 3490 |
| CTLA-4⁺ Treg | 107 | 95 | 254 | 205 | 92 | 3S |
| PD-1⁺ Treg | 229 | 232 | 664 | 557 | 494 | 152 |
| CTLA-4⁺ CD8⁺ Treg | 8 | 9 | 16 | 11 | 6 | 5 |
| PD-1⁺ CD8⁺ cells | 2196 | 2689 | 1868 | 989 | 1858 | 794 |

### [Table 24]

**Table 24. Number of cells (number per 10 mg of tissue) - 72 hr**

| Type of cell | Control | | Composition 20 | | HypoThermosol | |
|---|---|---|---|---|---|---|
| | Average | SD | Average | SD | Average | SD |
| Living cells | 95833 | 40162 | 231754 | 86163 | 112922 | 16571 |
| Lymphocytes | 25204 | 17928 | 90853 | 38866 | 22561 | 455 |
| CD3⁺ cells | 13706 | 13052 | 47162 | 22921 | 7897 | 1078 |
| CD4⁺ cells | 7152 | 6501 | 24223 | 11495 | 4145 | 1406 |
| Treg | 1368 | 1251 | 4751 | 1608 | 1042 | 73 |
| CD8⁺ cells | 5441 | 6017 | 18010 | 10522 | 1108 | 13 |
| CTLA-4⁺ Treg | 107 | 95 | 275 | 120 | 17 | 5 |
| PD-1⁺ Treg | 229 | 232 | 660 | 183 | 197 | 1 |
| CTLA-4⁺ CD8⁺ Treg | 8 | 9 | 13 | 7 | 1 | 7 |
| PD-1⁺ CD8⁺ cells | 2196 | 52689 | 6337 | 4616 | 279 | 56 |

### [Table 25]

**Table 25. Percentages (%) of each type of cell - 48 hr**

| Type of cell | Control | | Composition 20 | | HypoThermosol | |
|---|---|---|---|---|---|---|
| | Average | SD | Average | SD | Average | SD |
| CD3⁺ cells | 44.5 | 15.9 | 48.6 | 2.2 | 48.8 | 3.8 |
| CD4⁺ cells | 54.7 | 15.9 | 52.9 | 5.6 | 54.1 | 13.5 |
| Treg | 18.9 | 0.5 | 26.2 | 5.7 | 24.2 | 7.7 |
| CD8⁺ cells | 30.8 | 10.0 | 26.5 | 3.0 | 27.2 | 9.9 |
| CTLA-4⁺ Treg | 8.1 | 0.3 | 7.3 | 1.4 | 3.4 | 0.9 |
| PD-1⁺ Treg | 15.6 | 2.8 | 18.5 | 2.5 | 20.1 | 8.5 |
| CTLA-4⁺ CD8⁺ Treg | 0.2 | 0.1 | 0.2 | 0.1 | 0.1 | 0.0 |
| PD-1⁺CD8⁺ cells | 29.4 | 10.1 | 31.2 | 5.4 | 30.8 | 4.2 |

### [Table 26]

**Table 25. Percentages (%) of each type of cell - 72 hr**

| Type of cell | Control | | Composition 20 | | HypoThermosol | |
|---|---|---|---|---|---|---|
| | Average | SD | Average | SD | Average | SD |
| CD3⁺ cells | 44.5 | 15.9 | 50.3 | 3.7 | 34.9 | 4.1 |
| CD4⁺ cells | 54.7 | 3.1 | 51.8 | 0.8 | 51.0 | 10.8 |
| Treg | 18.9 | 0.5 | 21.3 | 3.5 | 27.8 | 7.7 |
| CD8⁺ cells | 30.8 | 10.0 | 35.8 | 4.9 | 14.3 | 1.8 |
| CTLA-4⁺ Treg | 8.1 | 0.3 | 5.6 | 0.6 | 1.6 | 0.6 |
| PD-1⁺ Treg | 15.6 | 2.8 | 14.3 | 0.9 | 18.5 | 5.5 |
| CTLA-4⁺ CD8⁺ Treg | 0.2 | 0.1 | 0.1 | 0.0 | 0.1 | 0.1 |
| PD-1⁺ CD8⁺ cells | 29.4 | 10.1 | 30.7 | 7.7 | 25.1 | 4.7 |

### (Embodiment 10: Performance Evaluation of the Composition According to the Present Disclosure Compared to Myeloid Lineage Cells)

The performance of the composition according to the present disclosure was evaluated compared to myeloid lineage cells. Here, myeloid cells are bone marrow-derived neutrophils, eosinophils, basophils, and precursor cells thereof. First, the components shown in Table 19 were dissolved in ultrapure water to prepare composition 18. Next, approximately 30 mg (3 pieces: approximately 10 mg/piece) of colon cancer-derived tumor tissue excised from a patient was immersed in approximately 15 mL of the composition and preserved at 0°C to 1°C. After 48 hours and 72 hours, the reagents listed in Table 27 were added and the cells were labeled. After labeling, the sample suspension solutions were washed and analyzed with a flow cytometer. The control was handled the same except that the excised tissue was immediately subjected to the enzyme treatment without preservation with a composition. The tests were carried out with n=3.

### [Table 27]

**Table 27. Reagents used for cell labeling (human-derived samples)**

| Reagent type | Product name (manufacturer) |
|---|---|
| Reagent for life-or-death determination | BD Horizon (trademark) Fixable Viability Stain 780 (BD) |
| Anti-human CD3 antibody | BD Pharmingen (trademark) Alex Fluor (registered trademark) 700 Mouse Anti-Human CD3 (UCHT1 (BD) |
| Anti-human CD14 antibody | CD14 Monoclonal Antibody (61D3), PE, eBioscience (trademark) (Invitrogen) |
| Anti-human HLA antibody | BD Pharmingen (trademark) FITC Mouse Anti-Human HLA-DR, DP, DQ (Tu39) (BD) |
| Anti-human CD11b antibody | BD Horizon (trademark) BV421 Mouse Anti-Human CD11b/MAC-1 (ICRF44) BD) |
| Anti-human CD11c antibody | BD Pharmingen (trademark) PE Mouse Anti-Human CD11c (B-ly6) (BD) |
| Anti-human CD33 antibody | Brilliant Violet 605 (trademark) anti-human CD33 Antibody (P67.6) (BioLegend) |
| Anti-human CD68 antibody | BD Pharmingen (trademark) Alexa Fluor (registered trademark) 647 Mouse Anti-Human CD68 (Y1/82A) (BD) |
| Anti-human CD163 antibody | BD OptiBuild (trademark) BV510 Mouse Anti-Human CD163 (GHI/61) (BD) |

The results of the flow cytometry were used to first identify living cells, then to analyze populations other than lymphocyte populations, and then to identify CD14-negative and HLA-negative or -low-expressing cells (CD14 HLAneg/low cells), CD14-positive and HLA-negative or -low-expressing cells (CD14⁺ HLAneg/low cells), and CD14- and HLA-positive cells (CD14⁺ HLA⁺ cells). Among all the cells, CD11b⁺ CD11c⁺ cells and CD11b⁺ CD33⁺ cells were further gated. In the CD14⁺ HLA⁺ cell population, CD68-negative and CD163-negative cells (CD68⁻CD163⁻ cells) and CD68-negative and CD163-positive cells (CD68⁻ CD163⁺ cells) were also gated.

The results are shown in Tables 28 and 29. At all times, a number of cells was detected which was equal to or greater than that in the control. Furthermore, the percentage of each type of cell was basically the same as the control. It has therefore been demonstrated that the analysis method according to the present disclosure can detect a greater number of living cells even in myeloid cells while maintaining the state of the cells.

### [Table 28]

**Table 28. Number of cells (number per 10 mg of tissue)**

| Type of cell | Control | | 48 hr | | 72 hr | |
|---|---|---|---|---|---|---|
| | Average | SD | Average | SD | Average | SD |
| Living cells | 25450 | 7492 | 52496 | 11884 | 60527 | 11712 |
| CD14⁻ HLAneg/low | 53603 | 29127 | 82054 | 14919 | 96060 | 13180 |
| CD11b⁺ CD11c⁺ | 3150 | 1427 | 5572 | 2013 | 3637 | 191 |
| CD11b⁺ CD33⁺ | 3463 | 1544 | 5785 | 2053 | 3772 | 196 |
| CD14⁺ HLAneg/low | 207 | 98 | 653 | 188 | 412 | 79 |
| CD11b⁺ CD11c⁺ | 163 | 81 | 523 | 152 | 348 | 74 |
| CD11b⁺ CD33⁺ | 144 | 78 | 419 | 99 | 271 | 59 |
| CD14⁺ HLA⁺ | 655 | 425 | 2038 | 814 | 1735 | 424 |
| CD11b⁺ CD11c⁺ | 603 | 409 | 1810 | 744 | 1541 | 457 |
| CD11b⁺ CD33⁺ | 603 | 409 | 1835 | 749 | 1557 | 445 |
| CD68⁻ CD163⁺ | 359 | 222 | 1137 | 475 | 983 | 274 |
| CD68⁻ CD163⁻ | 289 | 197 | 856 | 324 | 724 | 152 |

### [Table 29]

**Table 29. Percentages (%) of each type of cell**

| Type of cell | Control | | 48 hr | | 72 hr | |
|---|---|---|---|---|---|---|
| | Average | SD | Average | SD | Average | SD |
| Living cells | 29.8 | 5.6 | 33.0 | 1.2 | 32.8 | 6.4 |
| CD14⁻ HLAneg/low | 953 | 10.8 | 91.7 | 10.4 | 92.1 | 2.7 |
| CD11b⁺ CD11c⁺ | 6.1 | 1.2 | 6.7 | 2.1 | 3.8 | 0.4 |
| CD11b⁺ CD33⁺ | 6.7 | 11.3 | 7.0 | 2.1 | 4.0 | 0.4 |
| CD14⁺ HLAneg/low | 0.4 | 0.0 | 0.7 | 0.1 | 0.4 | 0.0 |
| CD11b⁺ CD11c⁺ | 78.0 | 2.4 | 80.1 | 0.9 | 84.4 | 20 |
| CD11b⁺ CD33⁺ | 67.4 | 15.4 | 65.4 | 4.1 | 65.4 | 26 |
| CD14⁺ HLA⁺ | 1.1 | 0.2 | 2.2 | 0.5 | 1.7 | 0.3 |
| CD11b⁺ CD11c⁺ | 90.2 | 3.1 | 88.5 | 1.4 | 87.7 | 4.8 |
| CD11b⁺ CD33⁺ | 90.2 | 3.0 | 89.8 | 1.0 | 88.8 | 3.7 |
| CD68⁻ CD163⁺ | 56.3 | 2.3 | 55.4 | 1,0 | 56.1 | 3.3 |
| CD68⁻ CD163⁻ | 42.6 | 2.2 | 42.4 | 1.1 | 42.3 | 3.3 |

## Claims

1. A composition for preserving a sample, including
albumin, and
lactic acid or a salt thereof,
wherein the sodium ion concentration of the composition is 50-300 mmol/L.

2. The composition as claimed in claim 1, wherein the albumin is included in an amount of 0.01-2% (w/v).

3. The composition as claimed in claim 1 or 2, wherein the lactic acid or salt thereof is included in an amount of 0.1-100 mmol/L.

4. The composition as claimed in any one of claims 1 to 3, wherein the albumin in bovine serum albumin (BSA).

5. The composition as claimed in any one of claims 1 to 4, wherein the lactic acid or salt thereof is sodium lactate.

6. The composition as claimed in any one of claims 1 to 5, including
0.01-10 mmol/L of potassium dihydrogen phosphate, and
0.01-50 mmol/L of disodium hydrogen phosphate.

7. The composition as claimed in any one of claims 1 to 6, wherein the composition includes a polysaccharide.

8. The composition as claimed in claim 7, wherein the polysaccharide is selected from the group consisting of trehalose, a fructooligosaccharide, indigestible dextrin, and a mixture thereof.

9. The composition as claimed in any one of claims 1 to 8, wherein the sample is cells, a tissue, or a mixture thereof.

10. The composition as claimed in any one of claims 1 to 9, wherein the sample is taken from a mammal.

11. The composition as claimed in claim 10, wherein the mammal is selected from the group consisting of human, dog, rat, and mouse.

12. A sample preservation method, wherein a sample is immersed in the composition as claimed in any one of claims 1 to 11.

13. The preservation method as claimed in claim 12, wherein a preservation temperature is 0°C to 4°, and the composition is in a non-frozen state.

14. A method for preparing a sample suspension solution including a target substance from a sample, including
a) a step of providing the composition as claimed in any one of claims 1 to 11,
b) a step of immersing the sample in the composition, and
c) a step of preparing the sample suspension solution by treating the sample with an enzyme.

15. The method as claimed in claim 14, wherein the enzyme is at least one selected from the group consisting of collagenase, elastase, hyaluronidase, dispase, glycosidase, deoxyribonuclease, and trypsin.

16. The method as claimed in claim 14 or 15, wherein the target substance is cells.

17. The method as claimed in claim 15, wherein the cells include living cells.

18. The method as claimed in claim 16 or 17, wherein the cells are tumor infiltrating immune cells.

19. A method for analyzing a target substance in a sample, including
a) a step of providing the composition as claimed in any one of claims 1 to 11,
b) a step of immersing the sample in the composition,
c) a step of preparing the sample suspension solution by treating the sample with an enzyme, and
d) a step of analyzing the target substance in the sample suspension solution.

20. The method as claimed in claim 19, wherein step d) is carried out by at least one selected from the group consisting of, flow cytometry, mass cytometry, gene analysis including single-cell gene analysis, imaging, an immunoassay, mass spectrometry, spectroscopic analysis, and combinations thereof.
